# EUROPÄISCHE PATENTSCHRIFT

(11) **EP 1 793 755 B1**
(45) Veröffentlichungstag und Bekanntmachung des Hinweises auf die Patenterteilung: **12.09.2012**
(21) Anmeldenummer: 05761125.3
(22) Anmeldetag: 15.07.2005
(51) Int. Cl.: A61B 18/14, A61B 17/94

(54) **ELEKTROCHIRURGISCHES INSTRUMENT**
ELECTROSURGICAL INSTRUMENT
INSTRUMENT ELECTROCHIRURGICAL

(30) Priorität: 11.08.2004 DE 102004039053; 18.11.2004 DE 102004055669
(43) Veröffentlichungstag der Anmeldung: 13.06.2007
(73) Patentinhaber: ERBE Elektromedizin GmbH, 72072 Tübingen (DE)
(72) Erfinder: HAFNER, Dieter, 72070 Tübingen (DE)
(74) Vertreter: Bohnenberger, Johannes
(86) Internationale Anmeldenummer: PCT/EP2005/007737
(87) Internationale Veröffentlichungsnummer: WO 2006/018086

(56) Entgegenhaltungen:
- US-A- 5 443 463
- US-A- 5 797 938
- US-A- 5 944 718
- US-A1- 2004 049 185
- US-A1- 2004 116 979

## Beschreibung

Die Erfindung betrifft ein elektrochirurgisches Instrument nach dem Oberbegriff des Patentanspruches 1, wie schon aus US 5443 463 bekannt.

Elektrochirurgische Instrumente werden seit vielen Jahren in der Hochfrequenz-Chirurgie eingesetzt, um biologisches Gewebe insbesondere zu koagulieren, aber auch zu schneiden. Bei einer Koagulation wird ein hochfrequenter Strom durch das zu behandelnde Gewebe geleitet, so dass sich dieses aufgrund Eiweißkoagulation und Dehydratation verändert. Das Gewebe zieht sich dabei derart zusammen, dass die Gefäße verschlossen und Blutungen gestillt werden. Nach erfolgter Koagulation ist das Gewebe beispielsweise mittels eines mechanisch arbeitenden Schneidinstruments durchtrennbar.

Elektrochirurgische Vorgänge sind sowohl monopolar als auch bipolar durchführbar. Bei der monopolaren Technik weist das elektrochüurgische Instrument nur eine einzige Stromzuführung auf, das zu behandelnde Gewebe (bzw. ein Patient) ist demgemäß auf das andere Potential zu legen. Immer mehr an Bedeutung gewinnen jedoch bipolare Instrumente, die mit zwei voneinander elektrisch isolierten Abschnitten ausgebildet sind. Der Stromweg zwischen den Elektrodenteilen ist damit kalkulierbar und verläuft nicht weite Strecken durch den Körper des Patienten. Damit reduziert sich die Beeinflussung von beispielsweise Herzschrittmachern oder sonstigen Geräten, die während der Operation an dem Patienten angeschlossen sind.

Bipolare Koagulationsinstrumente weisen im Wesentlichen zwei gelenkig miteinander verbundene Branchen auf, an deren proximalen Enden Griffeinrichtungen zur Handhabung der Branchen vorgesehen sind. An distalen Enden der Branchen befinden sich Elektrodenteile mit Koagulationsflächen zum Fassen von Gewebe und zum Durchleiten des Koagulationsstromes durch das Gewebe. Der von einem HF-Generator gelieferte HF-Strom wird dazu über Stromzuführungseinrichtungen zu den Elektrodenteilen des bipolaren Instruments geleitet.

Bekannte bipolare Koagulationsinstrumente weisen an den Elektrodenteilen oftmals Durchlassbereiche auf, die einen Führungsspalt für ein Schneidinstrument ausbilden. Das heißt, die Elektrodenteile sind zumindest partiell geteilt, so dass an dem zwischen den Elektrodenteilen eingeklemmten Gewebe das Schneidinstrument ansetzbar ist. Der Führungsspalt ermöglicht demgemäß den Zugang des Schneidinstruments an das Gewebe, während es zwischen den Elektrodenteilen des Koagulationsinstruments arretiert ist. Außerdem ist der Führungsspalt dafür vorgesehen, das Schneidinstrument zu führen, um so einen präzisen Schnitt an dem Gewebe zu gewährleisten. Dies ist insbesondere für mechanisch zu bedienende Schneidinstrumente von Vorteil.

Ein derartiges Instrument ist beispielsweise aus der US 2003/0229344 A1 bekannt. Es ist eine bipolare Zange gezeigt, bei der eine Effektoreinheit und insbesondere deren elektrisch leitfähige Bereiche Schlitze aufweisen, um einen Zugang eines Schneidinstruments an einem in der Effektoreinheit eingeklemmten Gewebe zu ermöglichen. Die Schlitze sind derart ausgeführt, dass Koagulationsflächen der Elektrodenteile so wenig wie möglich unterbrochen werden. Bei dieser Ausgestaltung der Schlitze ist deren Wiederaufbereitung, d. h. deren Reinigung nur mit großem Aufwand möglich, weil die Zugänglichkeit in die Schlitze erschwert ist.

Um diesem Problem entgegenzuwirken, sehen andere bekannte Instrumente einen sehr breiten Schlitz bzw. Führungsspalt vor. Dabei wird eine erhebliche Verkleinerung der Koagulationsflächen bzw. eine unzureichende Führung insbesondere mechanischer Schneidinstrumente in Kauf genommen.

Auch werden bekannte Instrumente als Einweg-Lösungen angeboten, um eine Reinigung zu umgehen. Damit sind erhebliche Kosten verbunden.

Der Erfindung liegt daher die Aufgabe zugrunde, ein elektrochirurgisches Instrument zum Koagulieren der eingangs genannten Art dahin gehend weiterzubilden, dass ein Durchlassbereich an mindestens einem Elektrodenteil eine optimale Führung eines Schneidinstruments dauerhaft gewährleistet.

Diese Aufgabe wird durch ein elektrochirurgisches Instrument nach Patentanspruch 1 gelöste.

Insbesondere wird die Aufgabe durch ein elektrochirurgisches Instrument gelöst, das zwei gelenkig miteinander verbundene Branchen umfasst, die entsprechend einem Schneid- oder Klemmwerkzeug betätigbar sind. Ferner umfasst das Instrument einander gegenüberliegende Elektrodenteile mit Koagulationsflächen an distalen Enden der Branchen zum Fassen von einem Gefäß oder Gewebe und zum Durchleiten eines Koagulationsstromes durch das Gefäß oder Gewebe zu dessen Koagulation, wobei mindestens ein Elektrodenteil einen Durchlassbereich als Führungsspalt für ein Schneidinstrument aufweist, so dass das mindestens eine Elektrodenteil in mindestens zwei Bereiche aufgeteilt und an dem gefassten Gefäß oder Gewebe das Schneidinstrument zum Durchführen eines Schneidvorgangs ansetzbar ist. Des Weiteren sind Stromzuführungseinrichtungen zum Zuführen des Koagulationsstromes zu den Elektrodenteilen von einem HF-Generator vorgesehen. Die mindestens zwei Bereiche des mindestens einen Elektrodenteils weisen jeweils sich gegenüberliegende, sich in Richtung der Koagulationsflächen verjüngend zueinander angeordnete Teilungsflächen auf.

Ein wesentlicher Punkt der Erfindung liegt darin, dass sich der Führungsspalt in von einem Schneidbereich zwischen den Elektrodenteilen wegzeigender Richtung an beiden Elektrodenteilen aufweitet. Damit ist in unmittelbarer Nähe zum Schneidbereich aufgrund der verjüngten Ausgestaltung des Führungsspaltes eine präzise Führung des Schneidinstruments möglich und eine Koagulationszone der Elektrodenteile nur unwesentlich unterbrochen. Gleichzeitig sind übrige Bereiche des Führungsspaltes leicht zugänglich und damit auch leicht zu reinigen. Soll der Führungsspalt nachbearbeitet, also z. B. eine Beschichtung aufgetragen werden, so ist dies aufgrund der besseren Zugänglichkeit einfach zu bewerkstelligen.

In einer ersten bevorzugten Ausführungsform sind die Durchlassbereiche an den gegenüberliegenden Elektrodenteilen vorgesehen, wobei diese bei zusammengeführten Branchen im Wesentlichen fluchtend aneinander grenzen. Ist nur ein Durchlassbereich an einem Elektrodenteil ausgebildet, so eignet sich dieser insbesondere dafür, das Gewebe beispielsweise mittels eines chirurgischen Messers zu durchtrennen, wobei das Gewebe auf dem gegenüberliegenden Elektrodenteil vollständig, in gespanntem Zustand, aufliegt. Sind an beiden Elektrodenteilen Durchlassbereiche vorgesehen, so ist beispielsweise eine chirurgische Schere an dem koagulierten Gewebe ansetzbar und dieses auf einfache Weise zu durchtrennen. Zur Ausführung eines gut kalkulierbaren Schnittes sind die Durchlassbereiche vorzugsweise in den mittleren Abschnitten der Elektrodenteile angeordnet.

In einer weiteren bevorzugten Ausführungsform ist das Schneidinstrument mit dem elektrochirurgischen Instrument verbunden ausgebildet. Beispielsweise befindet sich das Schneidinstrument innerhalb einer der Branchen und kann bei Bedarf in eine Schneidposition gebracht werden. Damit ist ein Instrumentenwechsel vermeidbar, so dass ein Operationsverlauf nicht unterbrochen werden muss.

Bei in das Koagulationsinstrument integriertem Schneidinstrument sind vorzugsweise beide Elektrodenteile mit dem Durchlassbereich ausgebildet, damit das Schneidinstrument das Gewebe ungehindert erreichen kann.

Ist das Schneidinstrument nicht integrativ mit dem elektrochirurgischen Instrument ausgebildet, so ist der Führungsspalt derart auszulegen, dass ein von außen kommendes Schneidinstrument bei hinreichend genauer Führung an dem vorgespannten Gewebe ansetzbar ist.

Eine vorteilhafte Ausführungsform sieht vor, dass das Schneidinstrument mechanisch und/oder elektrisch betätigbar ist. So kann beispielsweise eine an einem Schaft ausgebildete Klinge an dem elektxochirurgischen Instrument vorgesehen sein, die während der Koagulation in der Branche untergebracht ist und für den Schneidvorgang an das Gewebe herangeführt wird. Das Positionieren der Klinge oder eines sonstigen Schneidinstruments und auch ein Vorschub können hierbei selbsttätig erfolgen oder auch von dem Chirurgen mechanisch durchgeführt werden.

Eine erfindungsgemäße Lösung sieht vor, dass das Schneidinstrument zum Schneiden mittels eines HF-Stromes ausgebildet und mit einer Steuerungseinheit verbunden ist, so dass der Schneidstrom in Abhängigkeit von Operationsphasen zuführbar ist. Der Operateur kann dann den Schneidvorgang derart steuern, dass dieser selbsttätig und optimiert abläuft.

In einer bevorzugten Ausführungsform ist vorgesehen, dass die Elektrodenteile jeweils mindestens einen Spannbereich aufweisen, derart, dass beim Einklemmen des Gewebes dieses zwischen den Elektrodenteilen vorgespannt wird und an dem vorgespannten Gewebe der Schneidvorgang mittels des Schneidinstruments durchführbar ist. Das unter Spannung stehende Gewebe ist dann mittels des Schneidinstruments, insbesondere mittels eines mechanischen Schneidinstruments, leichter zu schneiden, weil sich Fasern des Gewebes quer zu einer Schneidrichtung ausrichten und das Gewebe dabei dünner wird. Damit ist eine an dem vorgespannten Gewebe zu dessen vollständiger Durchtrennung aufzubringende Kraft erheblich reduziert und einer mechanischen Belastung des Schneidinstruments, insbesondere einem Verschleiß von Schneidabschnitten wird entgegengewirkt. Auch ist der Schneidvorgang durch den Operateur leichter zu bewerkstelligen und das Instrument einfacher zu handhaben. Aufgrund der sich zu dem Schneidbereich hin verjüngenden Teilungsflächen der Elektrodenteile kann insbesondere ein mechanisch zu bedienendes Schneidinstrument besonders leicht an dem Führungsspalt angesetzt werden.

In einer bevorzugten Ausführungsform ist einer der Spannbereiche mindestens in einem ersten mittleren Abschnitt konvex, der ihm gegenüberliegende Spannbereich mindestens in einem zweiten mittleren Abschnitt konkav gekrümmt. Damit passen die Spannbereiche bei einem Zusammenführen der Branchen im Wesentlichen formschlüssig ineinander. Durch die gekrümmten Spannbereiche wird auf einfachste Art ein Spannen des Gewebes ermöglicht, weil dieses durch die gekrümmten Bereiche beidseitig in Richtung deren Endbereiche gezogen, d. h. gestreckt wird. Durch den Formschluss ist das Gewebe dann zwischen den Branchen in gespanntem Zustand sicher arretiert.

Die Begriffe "konvex" und "konkav" sind in diesem Zusammenhang nicht nur als im Bogen gerundet zu verstehen. Vielmehr ist mit den Termini jede Art von Erhabenheit bzw. Einbuchtung zu verstehen, also z. B. auch eine dachförmige Erhabenheit und dementsprechend eine V-förmige Einbuchtung.

In einer weiteren bevorzugten Ausführungsform ist einer der Spannbereiche mindestens in dem ersten mittleren Abschnitt konvex, der ihm gegenüberliegende Spannbereich mindestens in dem zweiten mittleren Abschnitt konkav gekrümmt. Dabei ist ein Krümmungsradius des konkav gekrümmten Spannbereichs mindestens im zweiten mittleren Abschnitt größer als ein Krümmungsradius des konvex gekrümmten Spannbereichs in dem ersten mittleren Abschnitt. Die Krümmungen verlaufen um Längsachsen der distalen Enden derart, dass das zwischen den distalen Enden gehaltene und senkrecht zu den Längsachsen verlaufende Gewebe mit zu dem ersten und zweiten mittleren Abschnitt hin steigender Pressung gehalten wird. Durch die unterschiedlich gekrümmte Ausgestaltung der Koagulationsflächen kann eine Berührung der Koagulationsflächen mathematisch betrachtet nur an deren Scheitellinien erfolgen. Das heißt, zwischen den Koagulationsflächen ist ein Bereich maximaler Nähe ausgebildet, der sich symmetrisch um die Scheitellinien der Koagulationsflächen erstreckt. In diesem Bereich wird das Gewebe bei zusammengeführten Branchen aufgrund der erhöhten Pressung gegenüber übrigen Koagulationsbereichen besonders stark zusammengedrückt und damit sicher zwischen den Elektrodenteilen arretiert.

Vorteilhafterweise ist die glatte Geometrie einfach herstellbar, einem Anhaften von Gewebe während des Eingriffs wird entgegenwirkt und die Koagulationsflächen lassen sich leicht wiederaufbereiten und ggf. nachbearbeiten. Gleichzeitig wird an den Bereichen hoher Pressung, also hohen Drucks, bedingt durch die hohe Klemmkraft ein sicheres Verschließen des Gefäßes oder Gewebes erreicht.

Eine erfindungsgemäße Lösung sieht vor, dass an dem einen Spannbereich und/oder an dem gegenüberliegenden Spannbereich ein einen Spanneffekt unterstützendes Oberflächenprofil ausgebildet ist. Das Profil ist vorzugsweise an Endbereichen des jeweiligen Spannbereichs ausgebildet und bewegt das Gewebe zusätzlich in einer durch die Spannbereiche definierte Zugrichtung bzw. verhindert ein Zurückweichen des Gewebes entgegen dieser Zugrichtung.

Vorzugsweise ist das den Spanneffekt unterstützende Oberflächenprofil als Sägezahnprofil ausgebildet. Zähne des Profils können beispielsweise so angeordnet sein, dass sie während des Zusammenführens der Branchen immer weiter in das Gewebe greifen und dieses in Zugrichtung mitnehmen. Damit wird die Spannung im Gewebe deutlich erhöht. Es ist allerdings darauf zu achten, dass durch das Profil eine Verletzung des Gewebes vermieden wird, so dass die Zähne vorzugsweise als abgerundete Noppen ausgebildet sind.

Vorzugsweise ist das Profil derart ausgestaltet, dass das Gewebe bei einem leichten Öffnen der Branchen durch das Profil in seiner gespannten Position gehalten wird. Das Profil fungiert demgemäß als eine Anordnung von Widerhaken.

In einer bevorzugten Ausführungsform ist das den Spanneffekt unterstützende Oberflächenprofil derart ausgebildet, dass zwischen den Elektrodenteilen mindestens eine Verengung vorgesehen ist. Dies ist insbesondere bei Elektrodenteilen mit gleichen Krümmungsradien zweckmäßig. Das heißt, die Koagulationsflächen der insbesondere mit gleichem Krümmungsradius ausgebildeten Elektrodenteile sind vorzugsweise an den beiden Endbereichen derart ausgestaltet, dass das Gewebe während des Zusammenführens der Branchen in Richtung der Endbereiche mitgenommen wird und bei zusammengeführten Branchen jeweils in einer Verengung gegenüber dem übrigen Bereich eingeklemmt ist. Die Verengung hat zusätzlich den Vorteil, dass die Koagulationsflächen im Wesentlichen glatt ausgebildet sein können und so leicht zu reinigen sind. Zudem wird die Verletzung des Gewebes aufgrund der glatten Oberfläche vermieden.

In einer vorteilhaften Ausführungsform ist an mindestens einer der Koagulationsflächen ein Isolationsabschnitt ausgebildet, so dass ein direkter elektrischer Kontakt zwischen den Koagulationsflächen vermeidbar ist. Aufgrund wärmeleitender Eigenschaften des Isolationsabschnittes wird auch an diesem eine Koagulation des Gewebes gewährleistet. Der Isolationsabschnitt ist je nach Ausgestaltung der Elektrodenteile an den Bereichen mindestens einer Koagulationsfläche vorzusehen, die der gegenüberliegenden Koagulationsfläche am nächsten sind. Dies ist insbesondere dann zu beachten, wenn die Spannbereiche und damit die Koagulationsflächen einen unterschiedlichen Krümmungsradius aufweisen. Vorzugsweise ist der Isolationsabschnitt dann an dem mittleren Bereich des Spannbereichs oder der Spannbereiche angeordnet und verhindert so einen Kurzschluss zwischen den Elektrodenteilen. Gleichzeitig wird der Spanneffekt durch den Isolationsabschnitt weiter begünstigt.

Ist der Isolationsabschnitt an den Bereichen mindestens einer Koagulationsfläche mit nächster Nähe zu der gegenüberliegenden Koagulationsfläche ausgebildet, kann er bündig mit der jeweiligen Koagulationsfläche abschließen. Der Flächenanteil der Koagulationsfläche, der den Bereich mit nächster Nähe zu der gegenüberliegenden Koagulationsfläche beschreibt, muss dann jedoch durchgängig aus isolierendem Material ausgebildet sein, so dass ein Kontakt zwischen den leitenden Bereichen der Koagulationsflächen vermieden wird. Bei den konvex bzw. konkav ausgebildeten, gegenüberliegenden Spannbereichen bzw. Koagulationsflächen mit unterschiedlichen Krümmungsradien, müsste der Isolationsabschnitt entlang einer Scheitellinie mindestens einer Koagulationsfläche angeordnet sein. Vorteilhafterweise ist der Isolationsabschnitt bei dieser Ausführungsform geschützt in dem jeweiligen Elektrodenteil und somit sicher vor Verschleiß untergebracht.

Alternativ ist es möglich, den Isolationsabschnitt derart auszubilden, dass dieser aus der jeweiligen Koagulationsfläche hervorsteht. In diesem Falle dient der Isolationsabschnitt nicht nur der Isolation, sondern auch dazu, das zu behandelnde Gewebe mehrfach zu knicken und so eine verbesserte Arretierung des Gewebes zwischen den distalen Enden des elektrochirurgischen Instruments zu erreichen.

In einer bevorzugten Ausführungsform ist der Isolationsabschnitt, d. h. der aus der jeweiligen Koagulationsfläche hervorstehende Isolationsabschnitt, aus mehreren Teilabschnitten ausgebildet. Dies ermöglicht eine besonders sichere Arretierung des Gewebes zwischen den Elektrodenteilen, weil das Gewebe mehrfach an Kanten des Isolationsabschnittes geknickt wird.

Eine erfindungsgemäße Lösung sieht vor, den Isolationsabschnitt selbst strukturiert auszubilden, um so eine optimale Arretierung des Gewebes zu erreichen.

Eine bevorzugte Ausführungsform sieht vor, den Isolationsabschnitt aus Keramik oder Diamant auszubilden. Vorteilhafterweise weisen Keramik und Diamant u. a. eine hohe Korrosionsbeständigkeit und eine hohe Verschleißfestigkeit gegenüber mechanischer Belastung auf.

In einer weiteren bevorzugten Ausführungsform ist der Isolationsabschnitt als das oder jedes, den oder jeden Spanneffekt unterstützendes Oberflächenprofil ausgebildet. Damit wird auf einfachste Weise sowohl der Kurzschluss zwischen den Elektrodenteilen vermieden, als auch das Spannen des Gewebes verstärkt.

Eine wischen den Elektrodenteilen einen Kurzschluss verhindernde Einrichtung kann beispielsweise auch an den Branchen vorgesehen sein. Ist an diesen z. B. ein Abstandshalter angeordnet, können die Branchen nicht vollständig zusammengeführt werden, ein Abstand zwischen den Elektrodenteilen bleibt bestehen.

Elektrochirurgische Instrumente dieser Art können beispielsweise für den Einsatz am eröffneten Körper ausgebildet sein. Das Prinzip der mit einem Spannbereich ausgebildeten Elektrodenteile ist jedoch auch für in der Endoskopie eingesetzte Instrumente anwendbar. Die an den Branchen befestigten Elektrodenteile und ggf. das Schneidinstrument sind dann beispielsweise über einen an einem Schaft befestigten Handgriff zu betätigen oder aber es ist eine Steuerungseinheit vorgesehen, so dass eine Betätigung der Elektrodenteile und/oder des Schneidinstruments über diese gesteuert wird. So ist das elektrochirurgische Instrument vorzugsweise als laparoskopische Instrument ausgebildet.

Weitere Ausführungsformen der Erfindung ergeben sich aus den Unteransprüchen.

Nachfolgend wird die Erfindung anhand von Ausführungsbeispielen beschrieben, die anhand der Abbildungen näher erläutert werden. Hierbei zeigen
- - Fig. 1: eine schematisch im Schnitt dargestellte Elektrodenanordnung in Vorderansieht gemäß einer ersten bevorzugten Ausführungsform;
- - Fig. 2: ein perspektivisch dargestelltes elektrochirurgisches Instrument mit einer erfindungsgemäßen Elektrodenanordnung in einer zweiten bevorzugten Ausführungsform;
- - Fig. 3: die schematisch im Schnitt dargestellte Elektrodenanordnung in Vorderansicht gemäß der zweiten bevorzugten Ausführungsform aus Fig. 2;
- - Fig. 4: eine schematisch im Schnitt dargestellte Elektrodenanordnung in Vorderansicht gemäß einer dritten bevorzugten Ausführungsform;
- - Fig. 5: eine schematisch im Schnitt dargestellte Elektrodenanordnung in Vorderansicht gemäß einer vierten bevorzugten Ausführungsform.

In der nachfolgenden Beschreibung werden für gleiche und gleich wirkende Teile dieselben Bezugsziffern verwendet.

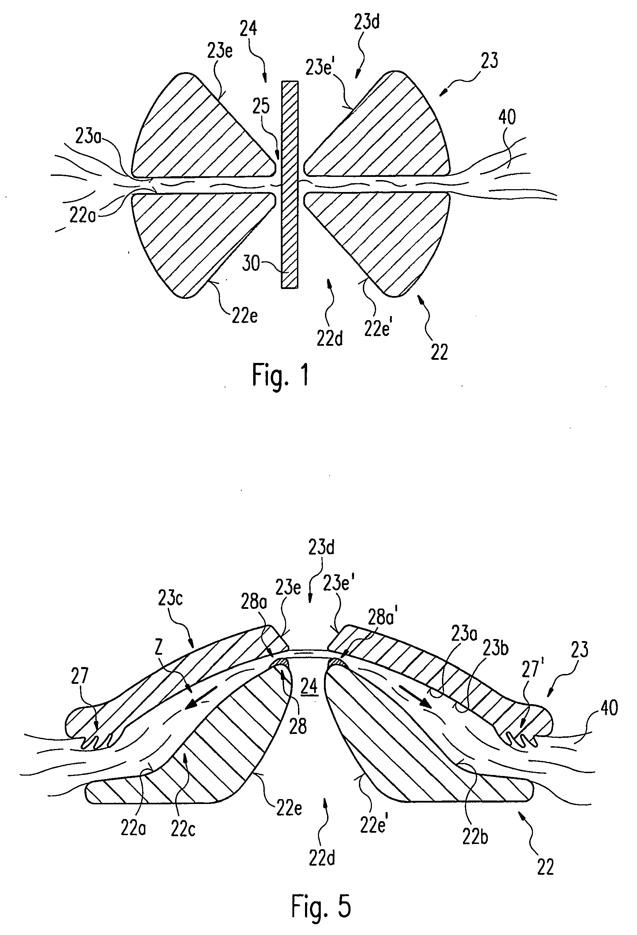

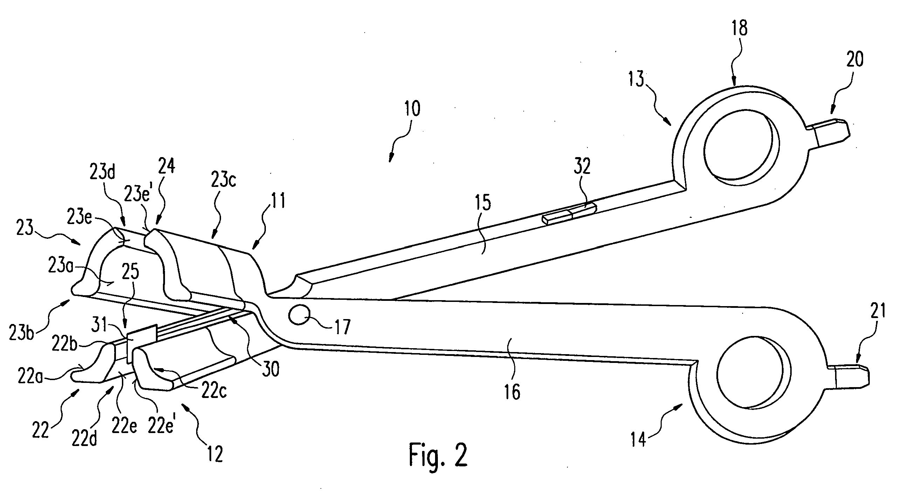

Fig. 1 zeigt eine schematisch im Schnitt dargestellte, vergrößerte Elektrodenanordnung in Vorderansicht gemäß einer ersten bevorzugten Ausführungsform. Die Elektrodenanordnung ist beispielsweise an einem elektrochirurgischen Instrument vorgesehen, wie es mit Fig. 2 näher beschrieben wird. Die Elektrodenteile 22, 23 weisen Durchlassbereiche 22d, 23d auf, die einen Führungsspalt 24 für ein Schneidinstrument 30 ausbilden. Aufgrund der Durchlassbereiche 22d, 23d bestehen die Elektrodenteile 22, 23 aus jeweils zwei Bereichen. An dem eingeklemmten Gewebe 40 ist demgemäß das Schneidinstrument 30 zum Durchführen eines Schneidvorgangs ansetzbar. Der Führungsspalt 24 ermöglicht zudem einen präzisen Schnitt an dem Gewebe 40, weil das Schneidinstrument 30 entlang dem Führungsspalt 24 führbar ist. Dies ist dann vorteilhaft, wenn das Schneidinstrument 30 mechanisch bedient wird. Wie aus der Abbildung ersichtlich, sind die Durchlassbereiche 22d, 23d fluchtend aneinander angeordnet, um den Schneidvorgang nicht zu behindern. Die mindestens zwei Bereiche der jeweiligen Elektrodenteile 22, 23 weisen jeweils sich gegenüberliegende, sich in Richtung von Koagulationsflächen 22a, 23a verjüngend zueinander angeordnete Teilungsflächen 22e, 22e', 23e, 23e' auf. Damit ist der Führungsspalt 24 in von einem Schneidbereich 25 zwischen den Elektrodenteilen 22, 23 wegzeigender Richtung an jedem Elektrodenteil 22, 23 aufgeweitet.

Aufgrund der verjüngten Ausgestaltung des Führungsspaltes 24 ist in unmittelbarer Nähe zum Schneidbereich 25 eine präzise Führung des Schneidinstruments 30 möglich und eine Koagulationszone der Elektrodenteile 22, 23 nur unwesentlich unterbrochen. Gleichzeitig sind übrige Bereiche des Führungsspaltes 24 leicht zugänglich und damit auch leicht zu reinigen. Soll der Führungsspalt 24 nachbearbeitet, also z. B. eine Beschichtung aufgetragen werden, so ist dies aufgrund der besseren Zugänglichkeit einfach zu bewerkstelligen.

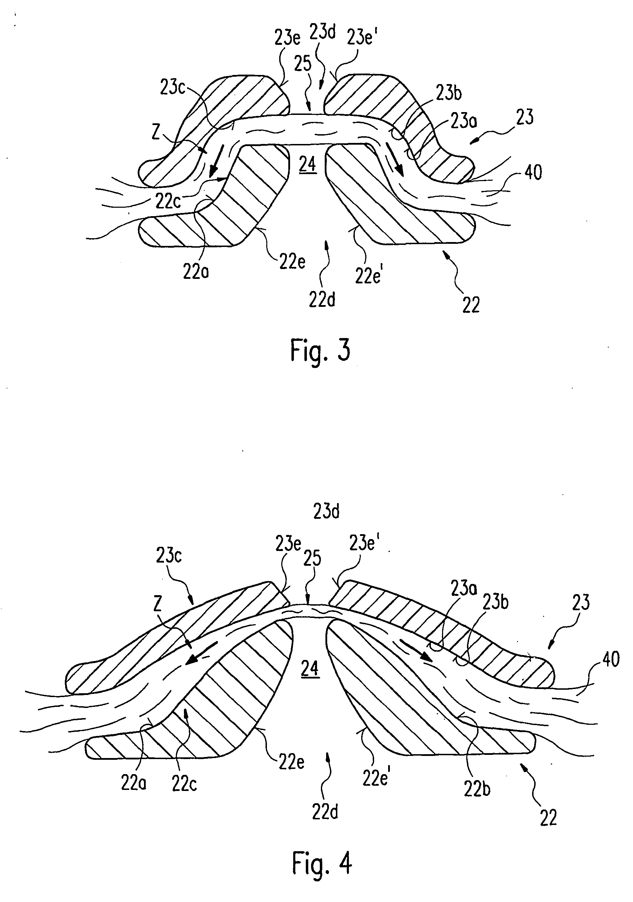

Fig. 2 zeigt ein perspektivisch dargestelltes elektrochirurgisches Instrument mit einer erfindungsgemäßen Elektrodenanordnung in einer zweiten bevorzugten Ausführungsform. Fig. 3 zeigt schematisch die im Schnitt dargestellte Elektrodenanordnung in Vorderansicht gemäß der zweiten bevorzugten Ausführungsform aus Fig. 2. Das Instrument 10 ist für einen Eingriff am eröffneten Körper ausgebildet. In der Abbildung sind mit den Bezugsziffern 15 und 16 zwei Branchen des elektrochirurgischen Instruments 10 bezeichnet. Die beiden Branchen 15, 16 sind über eine Achse 17 miteinander verbunden und um diese verschwenkbar. Sie weisen mit Elektrodenteilen 22, 23 versehene distale Enden 11, 12 auf, wobei sich die Elektrodenteile 22, 23 einander gegenüberliegen. Mit Hilfe der Elektrodenteile 22, 23, die Koagulationsflächen 22a, 23a aufweisen, lässt sich beispielsweise ein Gefäß oder Gewebe 40 fassen und durch Zuleitung von hochfrequentem Strom koagulieren. Ferner sind Griffteile 18, 19 vorgesehen, die an jeweilige proximale Enden 13, 14 der Branchen 15, 16 anschließen. Die proximalen Enden 13, 14 der Branchen 15, 16 enden jeweils in einem Stromanschlusselement bzw. einer Stromzuführungseinrichtung 20, 21 zum Anschließen des elektrochirurgischen Instruments 10 an einen (nicht dargestellten) HF-Generator, der eine HF-Spannung erzeugt, so dass der HF-Strom beispielsweise durch in dem Instrument 10 laufende elektrische Leitungen (nicht gezeigt) den Elektrodenteilen 22, 23 zugeführt werden kann.

Die Elektrodenanordnung entspricht zu großen Teilen der in Fig. 1 beschriebenen. Auch bei diesem Ausführungsbeispiel weisen die Elektrodenteile 22, 23 jeweils zwei Bereiche auf, die jeweils sich gegenüberliegende, sich in Richtung der Koagulationsflächen verjüngend zueinander angeordnete Teilungsflächen 22e, 22e', 23e, 23e' vorsehen. Damit ist auch hier ein Führungsspalt 24 in von einem Schneidbereich 25 zwischen den Elektrodenteilen 22, 23 wegzeigender Richtung an jedem Elektrodenteil 22, 23 aufgeweitet. Aufgrund des Führungsspaltes 24 kann das Gewebe 40 noch während der Arretierung zwischen den Elektrodenteilen 22, 23 mittels eines Schneidinstruments 30 geschnitten werden.

Die Elektrodenteile 22, 23 sind jedoch derart ausgebildet, dass sich ein Elektrodenteil 23 bei Zusammenführung der Branchen 15, 16 über das andere Elektrodenteil 22 stülpt, d. h., dieses abdeckt. Wie aus der Abbildung ersichtlich, sind die Elektrodenteile 22, 23 gekrümmt ausgebildet. Dabei weist ein Elektrodenteil 22 eine konvexe Krümmung 22b auf und das Elektrodenteil 23, das dem konvexen Elektrodenteil gegenüberliegt, weist eine konkave Krümmung 23b auf. Damit passen die Elektrodenteile 22, 23 bei zusammengeführten Branchen 15, 16 formschlüssig ineinander. Durch die gekrümmten Elektrodenteile 22, 23 wird das Gewebe 40 in Richtung von Endbereichen der Elektrodenteil 22, 23 gezogen, d. h. in einer Zugrichtung Z gestreckt. Die Elektrodenteile 22, 23 bilden demgemäß Spannbereiche 22c, 23c aus. Damit lässt sich das Gewebe 40 leichter schneiden, da sich Fasern des Gewebes 40 quer zu einer Schneidrichtung ausrichten und das Gewebe 40 dabei dünner wird. Durch den Formschluss ist das Gewebe 40 dann zwischen den Branchen 15, 16 in gespanntem Zustand fixiert. In diesem Ausführungsbeispiel sind die Elektrodenteile 22, 23 im Wesentlichen vollständig als Spannbereiche 22c, 23c ausgebildet. Alternativ ist es möglich, dass nur Abschnitte der Elektrodenteile Spannbereiche ausbilden.

Das Schneidinstrument 30 weist eine Klinge 31 an einem Schaft auf und ist während einer Koagulationsphase innerhalb der Branche 15 untergebracht. Für den Schneidvorgang lässt sich das Schneidinstrument 30 an dem bereits koagulierten Gewebe positionieren und zum Durchtrennen des Gewebes 40 mit einer definierten Vorschubgeschwindigkeit bewegen. Dies geschieht in diesem Ausführungsbeispiel beispielsweise durch eine (nicht gezeigte) das Schneidinstrument 30 ansteuernde Steuerungseinheit, die durch einen Fingerschalter 32 aktivierbar ist. Da das Schneidinstrument 30 in dem elektrochirurgischen Instrument 10 integriert ausgebildet ist, ist ein Instrumentenwechsel und damit eine Unterbrechung eines Operationsverlaufs vermeidbar.

Alternativ ist es möglich, das Schneidinstrument 30 mechanisch durch den Anwender zu betätigen. Der Chirurg kann dann die Klinge 31 bei Bedarf durch die Branche 15 an und durch das Gewebe schieben.

Ist an dem elektrochirurgischen Instrument keine Vorrichtung zum Schneiden des Gewebes vorgesehen, so ist der Führungsspalt derart auszulegen, dass ein von außen kommendes Schneidinstrument, z. B. eine chirurgische Schere, bei hinreichend genauer Führung an dem vorgespannten Gewebe ansetzbar ist.

In der praktischen Anwendung ist an dem elektrochirurgischen Instrument 10 ein Abstandshalter (nicht gezeigt) oder dergleichen zwischen den Elektrodenteilen 22, 23 einen Abstand haltende Vorrichtung ausgebildet, damit ein unmittelbarer Kontakt der Koagulationsflächen 22a, 23a der Elektrodenteile 22, 23 und damit ein Kurzschluss vermeidbar ist. Der Abstandshalter kann beispielsweise an einer der Branchen 15, 16 ausgebildet sein.

Alternativ ist es möglich, den Abstandshalter als Isolationsabschnitt an den Elektrodenteilen vorzusehen. Aufgrund wärmeleitender Eigenschaften des Isolationsabschnittes wird auch an diesem eine Koagulation gewährleistet.

Das in Fig. 2 gezeigte elektrochirurgische Instrument 10 ist, wie bereits oben erwähnt, für den Einsatz am eröffneten Körper ausgebildet. Das Prinzip der mit den sich verjüngenden Teilungsflächen ausgebildeten Elektrodenteile ist ebenso für die Endoskopie anzuwenden. Die an den Branchen befestigten Elektrodenteile und ggf. das Schneidinstrument sind dann beispielsweise über einen an einem Schaft befestigten Handgriff zu betätigen oder aber es ist eine Steuerungseinheit vorgesehen, so dass eine Betätigung der Elektrodenteile und/oder des Schneidinstruments über diese gesteuert wird.

Fig. 4 und 5 zeigen jeweils eine vergrößerte Vorderansicht einer Elektrodenanordnung im Schnitt in einer dritten und vierten Ausführungsform. Die Elektrodenteile 22, 23 entsprechen im Wesentlichen der Ausgestaltung der in Fig. 2 und 3 gezeigten. Auch weisen die Elektrodenteile 22, 23 ebenfalls jeweils zwei Bereiche auf, die jeweils sich gegenüberliegende, sich in Richtung von Koagulationsflächen 22a, 23a verjüngend zueinander angeordnete Teilungsflächen 22e, 22e', 23e, 23e' vorsehen. Damit ist auch hier ein Führungsspalt 24 in von einem Schneidbereich 25 zwischen den Elektrodenteilen 22, 23 wegzeigender Richtung an jedem Elektrodenteil 22, 23 aufgeweitet.

Bei diesen Ausführungsformen ist jeweils das Elektrodenteil 22 in einem ersten mittleren Abschnitt konvex gekrümmt, während das gegenüberliegende Elektrodenteil 23 in einem zweiten mittleren Abschnitt eine konkave Krümmung aufweist. Ein Krümmungsradius der konkav gekrümmten Koagulationsfläche 22a ist größer ist als ein Krümmungsradius der konvex gekrümmten Koagulationsfläche 22a. Die Krümmungen 22b, 23b verlaufen um Längsachsen der distalen Enden 11, 12 derart, dass ein zwischen den distalen Enden 11, 12 gehaltenes und senkrecht zu den Längsachsen verlaufendes Gefäß oder Gewebe 40 mit zu dem ersten und zweiten mittleren Abschnitt hin steigender Pressung gehalten wird. Aufgrund der Krümmungen 22b, 23b sind die Elektrodenteile 22, 23 auch bei diesen Ausführungsformen als Spannbereiche 22c, 23c ausgebildet. Das Gewebe 40 wird aufgrund der Spannbereiche 22c, 23c zu Endbereichen der Elektrodenteile 22, 23 hin in einer Zugrichtung Z gestreckt. Damit richten sich Fasern des Gewebes 40 quer zu einer Schneidrichtung aus, so dass das Gewebe 40 leichter geschnitten werden kann.

Fig. 5 unterscheidet sich im Wesentlichen von der in Fig. 4 gezeigten Elektrodenanordnung nur dadurch, dass ein aus zwei Teilabschnitten 28a, 28a' ausgebildeter, hervorstehender Isolationsabschnitt 28 unmittelbar benachbart einem Durchlassbereich 22d an dem durch den Durchlassbereich 22d in zwei Bereiche aufgeteilten, konvex ausgebildeten Elektrodenteil 22 vorgesehen ist. Vorzugsweise verlaufen die Teilabschnitte 28a, 28a' des Isolationsabschnitts 28 parallel einer Scheitellinie des Elektrodenteils 22. Damit wird ein Kurzschluss zwischen den Elektrodenteilen 22, 23 bei einem Zusammenführen derselben verhindert. Die Teilabschnitte 28a, 28a' des Isolationsabschnittes 28 unterstützen einerseits die Spannwirkung des Spannbereichs 22 und ermöglichen andererseits eine Knickung des eingespannten Gewebes 40. Damit ist dessen zuverlässige Arretierung zwischen den Elektrodenteilen 22, 23 gewährleistet.

Alternativ wäre es möglich, den Isolationsabschnitt derart an der Koagulationsfläche 22a auszubilden, dass er sich ebenfalls durchgängig entlang der Scheitellinie der Koagulationsfläche 22a erstreckt, im Wesentlichen aber bündig mit dieser abschließt. Der Isolationsabschnitt ist dann in die Koagulationsfläche 22a eingelegt. Dies ist deshalb möglich, weil der Isolationsabschnitt an dem ersten mittleren Abschnitt der Koagulationsfläche 22a vorgesehen wäre und so bei einem Zusammenführen der Branchen 15, 16 die gegenüberliegende Koagulationsfläche 23a zuerst und ausschließlich erreichen würde. Vorteilhafterweise wäre der Isolationsabschnitt bei dieser Ausführungsform geschützt in dem entsprechenden Elektrodenteil 22 und somit sicher vor Verschleiß untergebracht.

Vorzugsweise ist der Isolationsabschnitt 28 aus Keramik oder Diamant ausgebildet. Beide Materialien weisen u. a. eine hohe Korrosionsbeständigkeit und eine hohe Verschleißfestigkeit gegenüber mechanischer Belastung auf.

Das mit der konkaven Krümmung 23b ausgebildete Elektrodenteil 23 weist an den Endbereichen ein sägezahnartiges Profil 27, 27' auf. Die Zähne, können beispielsweise so angeordnet sein, dass sie während einem Zusammenführen der Branchen immer weiter in das Gewebe 40 greifen und dieses in Zugrichtung Z mitnehmen. Damit wird die Spannung im Gewebe 40 deutlich erhöht. Es ist allerdings darauf zu achten, dass durch das Profil 27, 27' eine Verletzung des Gewebes 40 vermieden wird, so dass die Zähne vorzugsweise als Noppen ausgebildet sind.

Vorzugsweise sind die Noppen derart angeordnet, dass das Gewebe 40 bei einem leichten Öffnen der Branchen durch das Profil 27, 27' in seiner gespannten Position gehalten wird. Das Profil 27, 27' fungiert demgemäß als eine Anordnung von Widerhaken.

Alternativ oder zusätzlich wäre es möglich, dass das den Spanneffekt unterstützende Oberflächenprofil derart ausgebildet, dass insbesondere zwischen den mit gleichem Krümmungsradius ausgebildeten Elektrodenteilen mindestens eine Verengung vorgesehen ist. Das heißt, die Koagulationsflächen der Elektrodenteile sind vorzugsweise an den beiden Endbereichen derart ausgestaltet, dass das Gewebe während des Zusammenführens der Branchen in Richtung der Endbereiche mitgenommen wird und bei zusammengeführten Branchen jeweils in einer Verengung gegenüber dem übrigen Bereich eingeklemmt ist. Die Verengung hat zusätzlich den Vorteil, dass die Koagulationsflächen im Wesentlichen glatt ausgebildet sein können und so leicht zu reinigen sind. Zudem wird die Verletzung des Gewebes aufgrund der glatten Oberfläche vermieden.

Vorteilhafterweise kann ein zwischen den Elektrodenflächen angeordneter Isolationsabschnitt als ein einen Spanneffekt der Spannbereiche unterstützendes Oberflächenprofil ausgebildet sein. Damit wird auf einfachste Weise sowohl die Vermeidung des Kurzschlusses zwischen den Elektrodenteilen vermieden, als auch das Spannen des Gewebes verstärkt.

An dieser Stelle sei darauf hingewiesen, dass alle oben beschriebenen Teile für sich alleine gesehen und in jeder Kombination, insbesondere die in den Zeichnungen dargestellten Details als erfindungswesentlich beansprucht werden. Abänderungen hiervon sind dem Fachmann geläufig.

### Bezugszeichenliste

- 10: Elektrochirurgisches Instrument
- 11: Distales Ende
- 12: Distales Ende
- 13: Proximales Ende
- 14: Proximales Ende
- 15: Branche
- 16: Branche
- 17: Achse
- 18: Griffteil
- 19: Griffteil
- 20: Stromanschlusselement, Stromzuführungseinrichtung
- 21: Stromanschlusselement, Stromzuführungseinrichtung
- 22: Elektrodenteil
- 22a: Koagulationsfläche
- 22b: Konvexe Krümmung
- 22c: Spannbereich
- 22d: Durchlassbereich
- 22e, 22e': Teilungsfläche
- 23: Elektrodenteil
- 23a: Koagulationsfläche
- 23b: Konkave Krümmung
- 23c: Spannbereich
- 23d: Durchlassbereich
- 23e, 23e': Teilungsfläche
- 24: Führungsspalt
- 25: Schneidbereich
- 27, 27': Profil
- 28: Isolationsabschnitt
- 28a, 28a': Teilabschnitt des Isolationsabschnitts
- 30: Schneidinstrument
- 31: Klinge
- 32: Fingerschalter
- 40: Gewebe, Gefäß
- Z: Zugrichtung

## Patentansprüche

1. Elektrochirurgisches Instrument mit
- zwei gelenkig miteinander verbundenen Branchen (15, 16), die entsprechend einem Schneid- oder Klemmwerkzeug betätigbar sind,
- einander gegenüberliegenden Elektrodenteilen (22, 23) mit Koagulationsflächen (22a, 23a) an distalen Enden (11, 12) der Branchen (15, 16) zum Fassen von einem Gefäß oder Gewebe (40) und zum Durchleiten eines
Koagulationsstromes durch das Gefäß oder Gewebe (40) zu dessen Koagulation,
wobei mindestens ein Elektrodenteil (22, 23) an der jeweiligen Branche (15, 16) einen Durchlassbereich (22d, 23d) als Führungsspalt (24) für ein Schneidinstrument (30) aufweist, so dass das mindestens eine Elektrodenteil (22, 23) in mindestens zwei Bereiche aufgeteilt und an dem gefassten Gefäß oder Gewebe (40) das Schneidinstrument (30) zum Durchführen eines Schneidvorgangs ansetzbar ist,
- Stromzuführungseinrichtungen (20, 21) zum Zuführen des Koagulationsstromes zu den Elektrodenteilen (22, 23) von einem HF-Generator,
**dadurch gekennzeichnet, dass**
die zwei Bereiche des einen Elektrodenteils (22, 23) an der jeweiligen Branche (15, 16) jeweils sich gegenüberliegende, sich in Richtung der Koagulationsflächen (22a, 23a) verjüngend zueinander angeordnete Teilungsflächen (22e, 22e', 23e, 23e') aufweisen.

2. Elektrochirurgisches Instrument nach Anspruch 1,
**dadurch gekennzeichnet, dass**
Durchlassbereiche (22d, 23d) an den gegenüberliegenden Elektrodenteilen (22, 23) vorgesehen sind, die bei zusammengeführten Branchen (15, 16) im Wesentlichen fluchtend aneinander grenzen.

3. Elektrochirurgisches Instrument nach Anspruch 1 oder 2,
**dadurch gekennzeichnet, dass**
das Schneidinstrument (30) mit dem elektrochirurgischen Instrument (10) verbunden ausgebildet ist.

4. Elektrochirurgisches Instrument nach einem der vorhergehenden Ansprüche,
**dadurch gekennzeichnet, dass**
das Schneidinstrument (30) mechanisch und/oder elektrisch betätigbar ist.

5. Elektrochirurgisches Instrument nach einem der vorhergehenden Ansprüche,
**dadurch gekennzeichnet, dass**
das Schneidinstrument (30) zum Schneiden mittels eines HF-Stromes ausgebildet und mit einer Steuerungseinheit verbunden ist, so dass der Schneidstrom in Abhängigkeit von Operationsphasen zuführbar ist.

6. Elektrochirurgisches Instrument einem der vorhergehenden Ansprüche,
**dadurch gekennzeichnet, dass**
die Elektrodenteile (22, 23) jeweils mindestens einen Spannbereich (22c, 23c) aufweisen, derart, dass beim Einklemmen des Gewebes (40) dieses zwischen den Elektrodenteilen (22, 23) vorgespannt wird und an dem vorgespannten Gewebe (40) der Schneidvorgang mittels des Schneidinstruments (30) durchführbar ist.

7. Elektrochirurgisches Instrument nach Anspruch 6,
**dadurch gekennzeichnet, dass**
einer der Spannbereiche (22c) mindestens in einem ersten mittleren Abschnitt konvex, der ihm gegenüberliegende Spannbereich (23c) mindesten in einem zweiten mittleren Abschnitt konkav gekrümmt ist, so dass bei einem Zusammenführen der Branchen (15, 16) die Spannbereiche (22c, 23c) im Wesentlichen formschlüssig ineinander passen.

8. Elektrochirurgisches Instrument nach Anspruch 6,
**dadurch gekennzeichnet, dass**
einer der Spannbereiche (22c) mindestens in dem ersten mittleren Abschnitt konvex, der ihm gegenüberliegende Spannbereich (23c) mindestens in dem zweiten mittleren Abschnitt konkav gekrümmt ist, wobei ein Krümmungsradius des konkav gekrümmten Spannbereichs (23c) mindestens im zweiten mittleren Abschnitt größer ist als ein Krümmungsradius des konvex gekrümmten Spannbereichs (22c) in dem ersten mittleren Abschnitt und wobei die Krümmungen (22b, 23b) um Längsachsen der distalen Enden (11, 12) derart verlaufen, dass das zwischen den distalen Enden (11, 12) gehaltene und senkrecht zu den Längsachsen verlaufende Gewebe (40) mit zu dem ersten und zweiten mittleren Abschnitt hin steigender Pressung gehalten wird.

9. Elektrochirurgisches Instrument nach einem der Ansprüche 6 bis 8,
**dadurch gekennzeichnet, dass**
an dem einen Spannbereich und/oder an dem gegenüberliegenden Spannbereich ein einen Spanneffekt unterstützendes Oberflächenprofil (27, 27') ausgebildet ist.

10. Elektrochirurgisches Instrument nach Anspruch 9,
**dadurch gekennzeichnet, dass**
das den Spanneffekt unterstützende Oberflächenprofil (27, 27') als Sägezahnprofil ausgebildet ist.

11. Elektrochirurgisches Instrument nach einem der Ansprüche 9 oder 10,
**dadurch gekennzeichnet, dass**
das den Spanneffekt unterstützende Oberflächenprofil (27, 27') derart ausgebildet ist, dass zwischen den Elektrodenteilen (22, 23) mindestens eine Verengung vorgesehen ist.

12. Elektrochirurgisches Instrument nach einem der vorhergehenden Ansprüche,
**dadurch gekennzeichnet, dass**
an mindestens einer Koagulationsfläche ein Isolationsabschnitt (28) ausgebildet ist, so dass ein direkter elektrischer Kontakt zwischen den Koagulationsflächen (22a, 23a) vermeidbar ist.

13. Elektrochirurgisches Instrument nach Anspruch 12,
**dadurch gekennzeichnet, dass**
der Isolationsabschnitt (28) aus mehreren Teilabschnitten ausgebildet ist.

14. Elektrochirurgisches Instrument nach Anspruch 12 oder 13,
**dadurch gekennzeichnet, dass**
der Isolationsabschnitt (28) strukturiert ausgebildet ist.

15. Elektrochirurgisches Instrument nach einem der Ansprüche 12 bis 14,
**dadurch gekennzeichnet, dass**
der Isolationsabschnitt (28) aus Keramik oder Diamant ausgebildet ist.

16. Elektrochirurgisches Instrument nach einem der Ansprüche 12 bis 15,
**dadurch gekennzeichnet, dass**
der Isolationsabschnitt (28) als das oder jedes, den oder jeden Spanneffekt unterstützendes Oberflächenprofil (27, 27') ausgebildet ist.

17. Elektrochirurgisches Instrument nach einem der vorhergehenden Ansprüche,
**dadurch gekennzeichnet, dass**
das Instrument als laparoskopisches Instrument ausgebildet ist.

## Claims

1. Electrosurgical instrument comprising
- two branches (15, 16) which are interconnected in a hinged manner and can be actuated in a corresponding manner to a cutting or clamping tool,
- electrode parts (22, 23) with coagulation areas (22a, 23a), situated opposite to one another at distal ends (11, 12) of the branches (15, 16), for gripping a vessel or tissue (40) and for passing coagulation current through the vessel or tissue (40) in order to coagulate the latter,
wherein at least one electrode part (22, 23) has a passage region (22d, 23d) on the respective branch (15, 16) as guide gap (24) for a cutting instrument (30) such that the at least one electrode part (22, 23) is subdivided into at least two regions and the cutting instrument (30) can be placed onto the gripped vessel or tissue (40) for carrying out the cutting process,
- current supply apparatuses (20, 21) for supplying the coagulation current to the electrode parts (22, 23) from an RF generator, **characterized in that**
on the respective branch (15, 16) the two regions of the one electrode part (22, 23) respectively have opposing dividing areas (22e, 22e', 23e, 23e') which are arranged tapering with respect to one another in the direction of the coagulation areas (22a, 23a).

2. Electrosurgical instrument according to Claim 1,
**characterized in that**
passage regions (22d, 23d) are provided on the opposing electrode parts (22, 23), which adjoin one another in a substantially flush fashion when the branches (15, 16) are brought together.

3. Electrosurgical instrument according to Claim 1 or 2,
**characterized in that**
the cutting instrument (30) is formed connected to the electrosurgical instrument (10).

4. Electrosurgical instrument according to one of the preceding claims,
**characterized in that**
the cutting instrument (30) can be actuated mechanically and/or electrically.

5. Electrosurgical instrument according to one of the preceding claims,
**characterized in that**
the cutting instrument (30) is formed for cutting by means of an RF current and connected to a control unit such that the cutting current can be supplied depending on operation phases.

6. Electrosurgical instrument according to one of the preceding claims,
**characterized in that**
the electrode parts (22, 23) respectively have at least one tensioning region (22c, 23c) such that, when the tissue (40) is clamped, the latter is pretensioned between the electrode parts (22, 23) and the cutting process can be carried out on the pretensioned tissue (40) by means of the cutting instrument (30).

7. Electrosurgical instrument according to Claim 6,
**characterized in that**
one of the tensioning regions (22c) has a convex curvature at least in a first central section, the tensioning region (23c) situated opposite thereto has a concave curvature at least in a second central section such that when the branches (15, 16) are brought together, the tensioning regions (22c, 23c) substantially have an interlocking fit into one another.

8. Electrosurgical instrument according to Claim 6,
**characterized in that**
one of the tensioning regions (22c) has a convex curvature at least in the first central section, the tensioning region (23c) situated opposite thereto has a concave curvature at least in the second central section, wherein a radius of curvature of the concavely curved tensioning region (23c) at least in the second central section is greater than a radius of curvature of the convexly curved tensioning region (22c) in the first central section and wherein the curves (22b, 23b) run about the longitudinal axes of the distal ends (11, 12) such that the tissue (40), which is held between the distal ends (11, 12) and runs perpendicularly to the longitudinal axes, is held with pressing that increases to the first and second central section.

9. Electrosurgical instrument according to one of Claims 6 to 8,
**characterized in that**
a surface profile (27, 27') which supports a tensioning effect is formed on the one tensioning region and/or on the opposing tensioning region.

10. Electrosurgical instrument according to Claim 9,
**characterized in that**
the surface profile (27, 27') supporting the tensioning effect is formed as saw-tooth profile.

11. Electrosurgical instrument according to one of Claims 9 or 10,
**characterized in that**
the surface profile (27, 27') supporting the tensioning effect is formed such that at least one narrowing is provided between the electrode parts (22, 23).

12. Electrosurgical instrument according to one of the preceding claims,
**characterized in that**
an insulation section (28) is formed on at least one coagulation area such that direct electrical contact can be avoided between the coagulation areas (22a, 23a).

13. Electrosurgical instrument according to Claim 12,
**characterized in that**
the insulation section (28) is formed from a plurality of partial sections.

14. Electrosurgical instrument according to Claim 12 or 13,
**characterized in that**
the insulation section (28) has a structured form.

15. Electrosurgical instrument according to one of Claims 12 to 14,
**characterized in that**
the insulation section (28) is formed from ceramic or diamond.

16. Electrosurgical instrument according to one of Claims 12 to 15,
**characterized in that**
the insulation section (28) is formed as the or every surface profile (27, 27') which supports the or every tensioning effect.

17. Electrosurgical instrument according to one of the preceding claims,
**characterized in that**
the instrument is embodied as laparoscopic instrument.

## Revendications

1. Instrument électrochirurgical doté :
de deux branches (15, 16) reliées entre elles de façon articulée et pouvant être actionnées conformément à un outil de coupe ou de serrage ;
de parties d'électrode (22, 23) opposées dotées de surfaces de coagulation (22a, 23a) au niveau des extrémités distales (11, 12) des branches (15, 16) pour saisir un vaisseau ou un tissu (40) et pour guider un courant de coagulation à travers le vaisseau ou le tissu (40) en vue de permettre sa coagulation ;
au moins une partie d'électrode (22, 23) présentant, au niveau de la branche respective (15, 16), une région de passage (22d, 23d) prenant la forme d'une fente de guidage (24) pour un instrument de coupe (30), de sorte que l'au moins une partie d'électrode (22, 23) est séparée en au moins deux régions et qu'un processus de coupe peut être réalisé au niveau du vaisseau ou du tissu (40) saisi à l'aide de l'instrument de coupe (30) ;
de dispositifs de conduction de courant (20, 21) servant à conduire le courant de coagulation jusqu'aux parties d'électrode (22, 23) en partant d'un générateur HF ;
**caractérisé en ce que** :
les deux régions d'une partie d'électrode (22, 23) comportent respectivement au niveau de la branche (15, 16) respective des surfaces de séparation (22e, 22e', 23e, 23e') opposées, disposées de façon à se rétrécir l'une par rapport à l'autre en direction des surfaces de coagulation (22a, 23a).

2. Instrument électrochirurgical selon la revendication 1, **caractérisé en ce que** les régions de passage (22d, 23d) sont prévues au niveau des parties d'électrode (22, 23) opposées se touchant les unes les autres pour l'essentiel de façon alignée lorsque les branches sont guidées conjointement (15, 16).

3. Instrument électrochirurgical selon la revendication 1 ou 2, **caractérisé en ce que** l'instrument de coupe (30) est réalisé de façon à être relié à l'instrument électrochirurgical (10).

4. Instrument électrochirurgical selon l'une quelconque des revendications précédentes, **caractérisé en ce que** l'instrument de coupe (30) peut être actionné de façon mécanique et/ou électrique.

5. Instrument électrochirurgical selon l'une quelconque des revendications précédentes, **caractérisé en ce que** l'instrument de coupe (30) est réalisé de façon à couper à l'aide d'un courant HF et est relié à une unité de commande, de sorte que le courant de coupe peut être conduit en fonction des phases de l'opération.

6. Instrument électrochirurgical selon l'une quelconque des revendications précédentes, **caractérisé en ce que** les parties d'électrode (22, 23) comportent respectivement au moins une région de serrage (22c, 23c), de telle sorte qu'en cas de pincement du tissu (40), celui-ci est précontraint entre les parties d'électrode (22, 23) et que le processus de coupe peut être réalisé au niveau du tissu (40) précontraint à l'aide de l'instrument de coupe (30).

7. Instrument électrochirurgical selon la revendication 6, **caractérisé en ce qu'**une des régions de serrage (22c) est coudée de façon convexe au moins dans une première section centrale située en vis-à-vis de la région de serrage (23c) coudée de façon concave au moins dans une deuxième section centrale, de sorte qu'en cas de guidage conjoint des branches (15, 16), les régions de serrage (22c, 23c) s'ajustent pour l'essentiel l'une dans l'autre par complémentarité de formes.

8. Instrument électrochirurgical selon la revendication 6, **caractérisé en ce qu'**une des régions de serrage (22c) est coudée de façon convexe au moins dans une première section centrale située en vis-à-vis de la région de serrage (23c) coudée de façon concave au moins dans une deuxième section centrale, un rayon de courbure de la région de serrage (23c) coudée de façon concave étant plus grand au moins dans la deuxième section centrale qu'un rayon de courbure de la région de serrage (22c) coudée de façon convexe dans la première section centrale et les courbures (22b, 23b) s'étendant autour des axes longitudinaux des extrémités distales (11, 12) de telle sorte que le tissu (40) maintenu entre les extrémités distales (11, 12) et s'étendant perpendiculairement aux axes longitudinaux est maintenu avec une pression croissante en direction de la première et de la deuxième section centrale.

9. Instrument électrochirurgical selon l'une quelconque des revendications 6 à 8, **caractérisé en ce qu'**un profil de surface (27, 27') contribuant à l'effet de serrage est réalisé au niveau d'une région de serrage et/ou de la région de serrage opposée.

10. Instrument électrochirurgical selon la revendication 9, **caractérisé en ce que** le profil de surface (27, 27') contribuant à l'effet de serrage prend la forme d'un profil en dents de scie.

11. Instrument électrochirurgical selon l'une quelconque des revendications 9 ou 10, **caractérisé en ce que** le profil de surface (27, 27') contribuant à l'effet de serrage est réalisé de telle sorte qu'au moins un rétrécissement est prévu entre les parties d'électrode (22, 23).

12. Instrument électrochirurgical selon l'une quelconque des revendications précédentes, **caractérisé en ce qu'**une section isolante (28) est réalisée au niveau d'au moins une surface de coagulation, de façon à éviter tout contact électrique direct entre les surfaces de coagulation (22a, 23a).

13. Instrument électrochirurgical selon la revendication 12, **caractérisé en ce que** la section isolante (28) est réalisée à partir de plusieurs sections partielles.

14. Instrument électrochirurgical selon la revendication 12 ou 13, **caractérisé en ce que** la section isolante (28) est réalisée de façon structurée.

15. Instrument électrochirurgical selon l'une quelconque des revendications 12 à 14, **caractérisé en ce que** la section isolante (28) est réalisée en céramique ou en diamant.

16. Instrument électrochirurgical selon l'une quelconque des revendications 12 à 15, **caractérisé en ce que** la section isolante (28) prend la forme du ou des profils de surface (27, 27') respectifs contribuant à l'effet de serrage.

17. Instrument électrochirurgical selon l'une quelconque des revendications précédentes, **caractérisé en ce que** l'instrument prend la forme d'un instrument laparoscopique.
